# FASCICULE DE BREVET EUROPEEN

(11) **EP 0 943 321 B1**
(45) Date de publication et mention de la délivrance du brevet: **11.04.2001**
(21) Numéro de dépôt: 98403311.8
(22) Date de dépôt: 28.12.1998
(51) Int. Cl.: A61K 7/42

(54) **Compositions cosmétiques pour la photoprotection de la peau et/ou des cheveux à base d'un mélange synergique de filtres et utilisations**
Kosmetische Mittel zum Lichtschutz der Haut und/oder der Haare auf Basis einer synergische Mischung von Filtern sowie ihren Verwendung
Sun-protection Cosmetic compositions for skin and/or hair based on a synergic mixture of filters and its uses

(30) Priorité: 27.02.1998 FR 9802416
(43) Date de publication de la demande: 22.09.1999
(73) Titulaire: L'OREAL, 75008 Paris (FR)
(72) Inventeur: Allard, Delphine, 92700 Colombes (FR); Gombert, Christelle, 95210 Saint Gratien (FR)
(74) Mandataire: Miszputen, Laurent

(56) Documents cités:
- EP-A- 0 742 003
- EP-A- 0 815 835
- EP-A- 0 824 909
- FR-A- 2 747 037
- GB-A- 2 286 774

## Description

La présente invention concerne de nouvelles compositions cosmétiques et/ou dermatologiques plus particulièrement destinées à la photoprotection de la peau et/ou des cheveux contre le rayonnement ultraviolet (compositions ci-après dénommées plus simplement compositions antisolaires), ainsi que leur utilisation dans l'application cosmétique susmentionnée. Plus précisément encore, elle concerne des compositions antisolaires comprenant, dans un support cosmétiquement acceptable, une association entre un premier filtre particulier, à savoir un dérivé de 1,3,5-triazine particulier avec au moins un deuxième filtre particulier convenablement sélectionné au sein des silicones benzotriazoles cette association conférant aux dites compositions, par un effet de synergie, des facteurs de protection solaire améliorés.

On sait que les radiations lumineuses de longueurs d'onde comprises entre 280 nm et 400 nm permettent le brunissement de l'épiderme humain et que les rayons de longueurs d'onde comprises entre 280 et 320 nm, connus sous la dénomination d'UV-B, provoquent des érythèmes et des brûlures cutanées qui peuvent nuire au développement du bronzage naturel ; ce rayonnement UV-B doit donc être filtré.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions phototoxiques ou photo-allergiques. Il est donc souhaitable de filtrer aussi le rayonnement UV-A.

De nombreuses compositions cosmétiques destinées à la photoprotection (UV-A et/ou UV-B) de la peau ont été proposées à ce jour.

Ces compositions antisolaires se présentent assez souvent sous la forme d'une émulsion de type huile-dans-eau (c'est à dire un support cosmétiquement acceptable constitué d'une phase continue dispersante aqueuse et d'une phase discontinue dispersée huileuse) qui contient, à des concentrations diverses, un ou plusieurs filtres organiques classiques, lipophiles et/ou hydrophiles, capables d'absorber sélectivement les rayonnements UV nocifs, ces filtres (et leurs quantités) étant sélectionnés en fonction du facteur de protection recherché (FPS) s'exprimant mathématiquement par le rapport du temps d'irradiation nécessaire pour atteindre le seuil érythématogène avec le filtre UV au temps nécessaire pour atteindre le seuil érythématogène sans filtre UV).

Or, à la suite d'importantes recherches menées dans le domaine de la photoprotection évoqué ci-dessus, la Demanderesse a découvert, de façon inattendue et surprenante, qu'une combinaison de deux filtres solaires particuliers et déjà connus en soi dans l'état de l'art, permettait, du fait d'un effet de synergie, d'obtenir des compositions antisolaires présentant des facteurs de protection solaire nettement améliorés, et en tous cas supérieurs à ceux qui peuvent être obtenus, à concentration égale de filtre et à nature identique de support, avec l'un ou l'autre des filtres utilisé seul.

Cette découverte est à la base de la présente invention.

Ainsi, conformément à l'un des objets de la présente invention, il est maintenant proposé de nouvelles compositions cosmétiques, en particulier antisolaires, qui sont essentiellement caractérisées par le fait qu'elles comprennent, dans un support cosmétiquement acceptable, (i) à titre de premier filtre au moins un dérivé de 1,3,5-triazine particulier que l'on définira plus en détail par la suite et (ii), à titre de deuxième filtre, un dérivé silicié à fonction benzotriazole particulier que l'on définira plus en détail par la suite ; lesdits premier et second filtres étant présents dans lesdites compositions dans une quantité efficace pour produire une activité synergique au niveau des facteurs de protection solaire conférés.

La présente invention a également pour objet l'utilisation de telles compositions comme, ou pour la fabrication de, compositions cosmétiques destinées à la protection de la peau et/ou des cheveux contre le rayonnement ultraviolet, en particulier le rayonnement solaire.

Un autre objet encore de la présente invention réside dans un procédé de traitement cosmétique pour la protection de la peau et/ou des cheveux contre le rayonnement ultraviolet, en particulier le rayonnement solaire, et qui consiste essentiellement à appliquer sur ces derniers une quantité efficace d'une composition conforme à l'invention.

D'autres caractéristiques, aspects et avantages de la présente invention apparaîtront à la lecture de la description détaillée qui va suivre.

Les dérivés de 1,3,5-triazine conformes à l'invention répondent à la formule générale suivante : 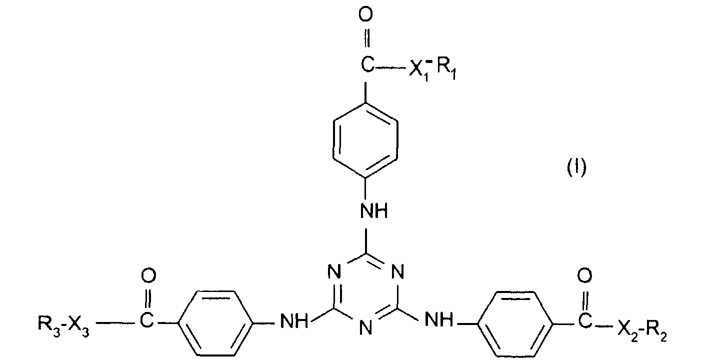 dans laquelle :
- X₁, X₂ et X₃, identiques ou différents, représentent l'oxygène ou un radical -NR-;
- les radicaux R, identiques ou différents, désignent l'hydrogène ou un radical alkyle en C₁-C₁₈, linéaire ou ramifié, un radical cycloalkyle en C₅-C₁₂ pouvant être substitué par un ou plusieurs radicaux alkyle en C₁-C₄ ;
- R₁, R₂ et R₃, identiques ou différents, sont choisis parmi : l'hydrogène; un métal alcalin; un radical ammonium éventuellement substitué par un ou plusieurs radicaux alkyle ou hydroxyalkyle ; un radical alkyle linéaire ou ramifié en C₁-C₁₈ ; un radical cycloalkyle en C₅-C₁₂ éventuellement substitué par un ou plusieurs radicaux alkyle en C₁-C₄ ; un radical polyoxyéthyléné comprenant de 1 à 6 unités d'oxyde d'éthylène et dont le groupe OH terminal est méthylé ; un radical de formule (II), (III) ou (IV) suivantes : 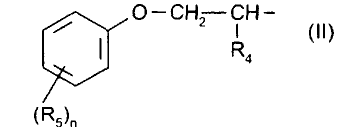 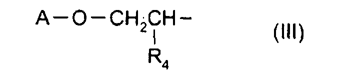 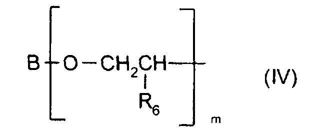
dans lesquelles :
- R₄ est l'hydrogène ou un radical méthyle;
- R₅ est un radical alkyle en C₁-C₉;
- n est un nombre entier allant de 0 à 3;
- m est un nombre entier allant de 1 à 10;
- A est un radical alkyle en C₄-C₈ ou un radical cycloalkyle en C₅-C₈;
- B est choisi parmi : un radical alkyle linéaire ou ramifié en C₁-C₈; un radical cycloalkyle en C₅-C₈; un radical aryle éventuellement substitué par un ou plusieurs radicaux alkyle en C₁-C₄;
- R₆ est l'hydrogène ou un radical méthyle.

Une première famille préférée de dérivés de 1,3,5-triazine est celle, notamment décrite dans le document EP-A-0517104 des 1,3,5-triazines répondant à la formule (I) ci-dessus et présentant l'ensemble des caractéristiques suivantes :
- X₁, X₂ et X₃ sont identiques et représentent l'oxygène ;
- R₁ est choisi parmi : un radical cycloalkyle en C₅-C₁₂ éventuellement substitué par un ou plusieurs radicaux alkyles en C₁-C₄; un radical de formule (II), (III) ou (IV) ci-dessus dans lesquelles :
- B est un radical alkyle en C₁-C₄;
- R₆ est le radical méthyle;
- R₂ et R₃, identiques ou différents, sont choisis parmi : l'hydrogène; un métal alcalin; un radical ammonium éventuellement substitué par un ou plusieurs radicaux alkyle ou hydroxyalkyle; un radical alkyle linéaire ou ramifié en C₁-C₁₈; un radical cycloalkyle en C₅-C₁₂ éventuellement substitué par un ou plusieurs radicaux alkyle en C₁-C₄; un radical de formule (II), (III) ou (IV) ci-dessus dans lesquelles :
- B est un radical alkyle en C₁-C₄;
- R₆ est le radical méthyle.

Une deuxième famille préférée de dérivés de 1,3,5-triazine selon l'invention est celle, notamment décrite dans le document EP-A-0570838 (dont les enseignements sont, pour ce qui touche à la définition même de ces produits, totalement inclus à titre de référence dans la présente description) des 1,3,5-triazines répondant à la formule (I) et présentant l'ensemble des caractéristiques suivantes :
- X₁ est l'oxygène ; X₂ est le radical -NH- ou l'oxygène
- X₃ est le radical -NH- ;
- R₃ est choisi parmi : un radical alkyle linéaire ou ramifié en C₁-C₁₈; un radical cycloalkyle en C₅-C₁₂ éventuellement substitué avec un ou plusieurs radicaux alkyles en C₁-C₄;
- R₁ est choisi parmi : l'hydrogène; un métal alcalin; un radical ammonium; un radical de formule (IV); un radical alkyle linéaire ou ramifié en C₁-C₁₈; un radical cycloalkyle en C₅-C₁₂ éventuellement substitué avec un ou plusieurs radicaux alkyles en C₁-C₄;
- si X₂ est le radical -NH-, alors R₂ est choisi parmi : un radical alkyle linéaire ou ramifié en C₁-C₁₈; un radical cycloalkyle en C₅-C₁₂ éventuellement substitué avec un ou plusieurs radicaux alkyles en C₁-C₄;
- si X₂ est l'oxygène, alors R₂ est choisi parmi l'hydrogène; un métal alcalin; un radical ammonium; un radical de formule (IV); un radical alkyle linéaire ou ramifié en C₁-C₁₈; un radical cycloalkyle en C₅-C₁₂ éventuellement substitué avec un ou plusieurs radicaux alkyles en C₁-C₄.

Une troisième famille préférée de dérivés de 1,3,5-triazine selon l'invention est celle, notamment décrite dans le document EP-A-0796851 dont les enseignements sont, pour ce qui touche à la définition même de ces produits, totalement inclus à titre de référence dans la présente description) des 1,3,5-triazines répondant à la formule (I) et présentant l'ensemble des caractéristiques suivantes :
- X₁, X₂ et X₃ désignent simultanément -NR- ;
- les radicaux R, identiques ou différents, désignent l'hydrogène ou un radical alkyle en C₁-C₁₈, linéaire ou ramifié, un radical cycloalkyle en C₅-C₁₂ pouvant être substitué par un ou plusieurs radicaux alkyle en C₁-C₄ ;
- R₁, R₂ et R₃, identiques ou différents, désignent l'hydrogène ou un radical R .

Une 1,3,5-triazine préférentielle est la 2,4,6-tris[p-(2'-éthylhexyl-1'-oxycarbonyl)anilino]-1,3,5-triazine qui est un filtre connu en soi, actif dans l'UV-B, se présentant sous une forme solide, et qui est vendu notamment sous la dénomination commerciale de "UVINUL T150" par la Société BASF. Ce produit répond à la formule suivante : 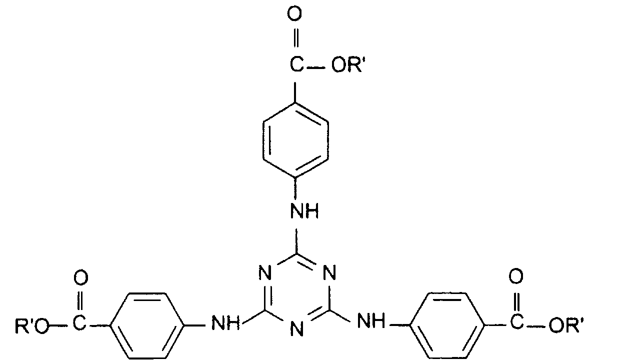 dans laquelle R' désigne un radical 2-éthyl hexyle.

Une 1,3,5-triazine selon l'invention particulièrement préférée est celle répondant à la formule suivante : 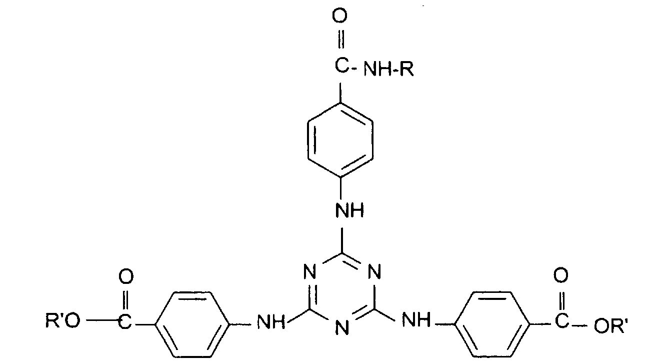 dans laquelle R' désigne un radical éthyl-2 hexyle et R désigne un radical tert- butyle.

Le ou les dérivés de triazine peuvent être présents dans les compositions selon l'invention à une teneur allant de 0,1 à 20%, de préférence de 0,2 à 15%, en poids, toujours par rapport au poids total de la composition.

Les dérivés siliciés à fonction benzotriazole utilisés dans la présente invention sont des silanes ou des siloxanes à fonction benzotriazole comprenant au moins une unité de formule (1) suivante :

O_{(3-a)/2}Si (R₇)ₐ - G (1)

dans laquelle :
- R₇ représente un radical alkyle en C₁-C₁₀ éventuellement halogéné ou un radical phényle ou un radical triméthylsilyloxy,
- a est un nombre entier choisi entre 0 et 3 inclusivement,
- et le symbole G désigne un radical monovalent lié directement à un atome de silicium, et qui répond à la formule (2) suivante : 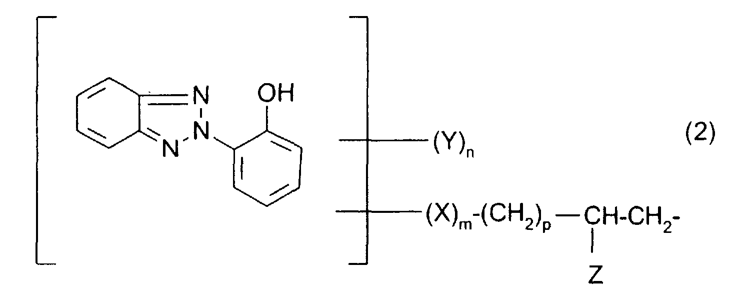
dans laquelle :
- Y, identiques ou différents, sont choisis parmi les radicaux alkyles en C₁-C₈, les halogènes et les radicaux alkoxy en C₁-C₄ étant entendu que, dans ce dernier cas, deux Y adjacents d'un même noyau aromatique peuvent former ensemble un groupement alkylidène dioxy dans lequel le groupe alkylidène contient de 1 à 2 atomes de carbone,
- X représente O ou NH,
- Z représente l'hydrogène ou un radical alkyle en C₁-C₄,
- n est un nombre entier compris entre 0 et 3 inclusivement,
- m est 0 ou 1,
- p représente un nombre entier compris entre 1 et 10, inclusivement.

Ces composés sont notamment décrits dans la demande de brevet EP-A-711778 au nom de la Demanderesse ainsi que dans la demande WO 94/06404 également au nom de la Demanderesse.

De préférence, les dérivés siliciés utilisés dans le cadre de la présente invention appartiennent à la famille générale des silicones benzotriazoles qui est décrite notamment dans WO 94/06404.

Une famille de silicones benzotriazoles convenant particulièrement bien à la réalisation de la présente invention est celle regroupant les composés répondant aux formules (5) ou (6) suivantes : 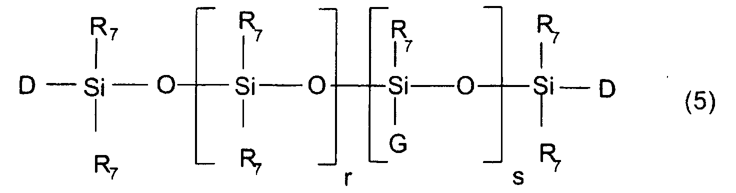 ou 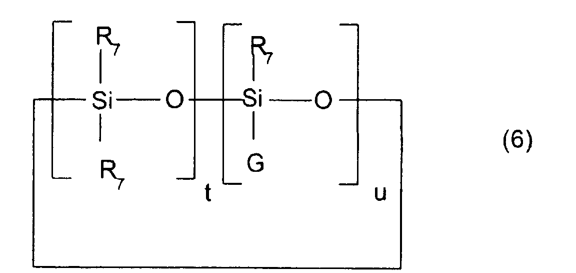 dans lesquelles :
- R₇, identiques ou différents, sont choisis parmi les radicaux alkyles en C₁-C₁₀, phényle, trifluoro-3,3,3 propyle et triméthylsilyloxy, au moins 80% en nombre des radicaux R₇ étant méthyle,
- D, identiques ou différents sont choisis parmi les radicaux R₇ et le radical G,
- r est un nombre entier compris entre 0 et 50 inclusivement, et s est un nombre entier compris entre 0 et 20 inclusivement, et si s = 0, au moins l'un des deux symboles D désigne G,
- u est un nombre entier compris entre 1 et 6 inclusivement, et t est un nombre entier compris entre 0 et 10 inclusivement, étant entendu que t + u est égal ou supérieur à 3,
- et le symbole G répond à la formule (2) ci-dessus.

Comme cela ressort de la formule (2) donnée ci-dessus, l'accrochage du chaînon -(X)ₘ-(CH₂)ₚ-CH(Z)-CH₂- sur le motif benzotriazole, qui assure donc le raccordement dudit motif benzotriazole à l'atome de silicium de la chaîne siliconée, peut, selon la présente invention, se faire dans toutes les positions disponibles offertes par les deux noyaux aromatiques du benzotriazole :

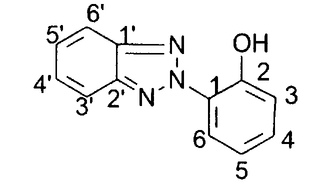

De préférence, cet accrochage se fait en position 3, 4, 5 (noyau aromatique portant la fonction hydroxy) ou 4' (noyau benzénique adjacent le cycle triazolé), et encore plus préférentiellement en position 3, 4 ou 5. Dans une forme préférée de réalisation de l'invention, l'accrochage se fait en position 3.

De même, l'accrochage du ou des motifs substituants Y peut se faire dans toutes les autres positions disponibles au sein du benzotriazole. Toutefois, de préférence, cet accrochage se fait en position 3, 4, 4', 5 et/ou 6. Dans une forme préférée de réalisation de l'invention, l'accrochage du motif Y se fait en position 5.

Dans les formules (5) et (6) ci-dessus, les radicaux alkyle peuvent être linéaires ou ramifiés et choisis notamment au sein des radicaux méthyle, éthyle, n-propyle, isopropyle, n-butyle, isobutyle, ter.-butyle, n-amyle, isoamyle, néopentyle, n-hexyle, n-heptyle, n-octyle, éthyl-2 hexyle et ter.-octyle. Les radicaux alkyle R₇ préférés selon l'invention sont les radicaux méthyle, éthyle, propyle, n-butyle, n-octyle et éthyl-2 hexyle. Encore plus préférentiellement, les radicaux R₇ sont tous des radicaux méthyle.

Parmi les composés de formules (5) ou (6) ci-dessus, on préfère mettre en oeuvre ceux répondant à la formule (5), c'est-à-dire des diorganosiloxanes à chaîne courte linéaire.

Parmi les composés de formules (5) ci-dessus, on préfère mettre en oeuvre ceux pour lesquels les radicaux D sont tous les deux des radicaux R₇

Parmi les diorganosiloxanes linéaires rentrant dans le cadre de la présente invention, on préfère plus particulièrement les dérivés statistiques ou bien définis à blocs présentant au moins l'une, et encore plus préférentiellement l'ensemble, des caractéristiques suivantes :
- D est un radical R₇
- R₇ est alkyle et encore plus préférentiellement est méthyle,
- r est compris entre 0 et 15 inclusivement ; s est compris entre 1 et 10 inclusivement,
- n est non nul, et de préférence égal à 1, et Y est alors choisi parmi méthyle, tert.-butyle ou alcoxy en C₁-C₄,
- Z est hydrogène ou méthyle,
- m=0, ou [m=1 et X=O]
- p est égal à 1.

Une famille de silicones benzotriazoles convenant particulièrement bien à l'invention est celle définie par la formule générale (7) suivante : 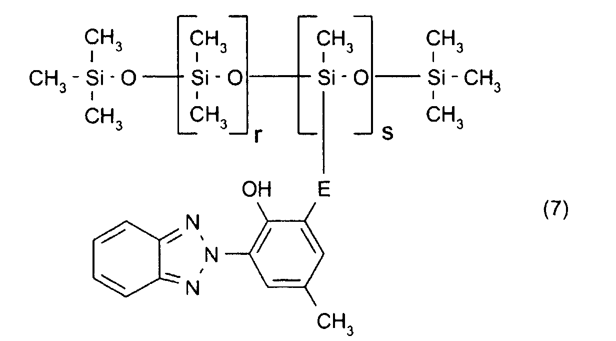 avec
0 ≤ r ≤ 10,
1 ≤ s ≤ 10,
et où E représente le radical divalent :

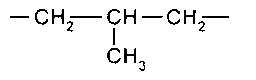

Dans une forme particulièrement préférée de réalisation de l'invention, la silicone benzotriazole est le composé (appelé composé (a) dans la suite du texte) répondant à la formule suivante :

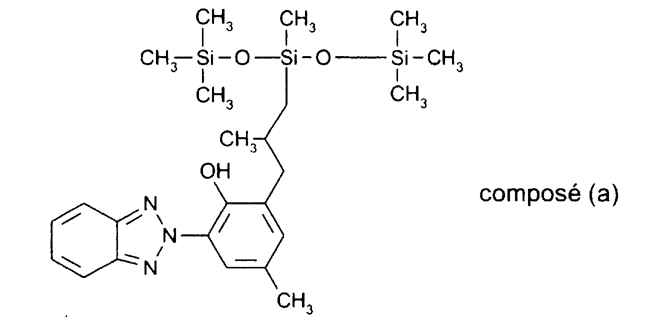

Des procédés convenant à la préparation des produits de formule (1), (5), (6) et (7) ci-dessus sont notamment décrits dans les brevets américains US 3,220, 972, US 3,697,473, US 4,340,709, US 4,316,033, US 4,328,346 et dans les demandes de brevet EP-A-0392883 et EP-A-0742003.

Le dérivé silicié à fonction benzotriazole peut être présent dans les compositions selon l'invention à des teneurs allant de 0,1 à 20%, de préférence allant de 0,2 à15%, en poids, toujours par rapport au poids total de la composition.

D'un point de vue pratique, les deux filtres ci-dessus, à savoir le dérivé de triazine et le dérivé silicié à motif benzotriazole, sont bien entendu de préférence tous deux présents dans la composition finale dans des proportions respectives choisies de manière telle que l'effet de synergie, au niveau du facteur de protection solaire conféré par l'association résultante, soit optimal.

Selon un mode préféré de réalisation de la présente invention, les compositions selon l'invention sont des émulsions de type huile-dans-eau.

Les compositions cosmétiques antisolaires selon l'invention peuvent bien entendu contenir un ou plusieurs filtres solaires complémentaires actifs dans l'UVA et/ou l'UVB (absorbeurs), hydrophiles ou lipophiles, autres bien sûr que les deux filtres mentionnés ci-avant. Ces filtres complémentaires peuvent être notamment choisis parmi les dérivés cinnamiques, les dérivés salicyliques, les dérivés du camphre, les dérivés de la benzophénone, les dérivés du dibenzoylméthane, les dérivés de β,β-diphénylacrylate, les dérivés de l'acide p-aminobenzoïque, les polymères filtres et silicones filtres autres que ceux à fonction benzotriazole notamment ceux décrits dans la demande WO-93/04665. D'autres exemples de filtres organiques sont donnés dans la demande de brevet EP-A-487404.

Les compositions selon l'invention peuvent également contenir des agents de bronzage et/ou de brunissage artificiels de la peau (agents autobronzants), tels que par exemple de la dihydroxyacétone (DHA).

Les compositions cosmétiques selon l'invention peuvent encore contenir des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demande de brevets EP-A-0518772 et EP-A-0518773.

Les compositions de l'invention peuvent comprendre en outre des adjuvants cosmétiques classiques notamment choisis parmi les corps gras, les solvants organiques, les épaississants ioniques ou non ioniques, les adoucissants, les antioxydants, les opacifiants, les stabilisants, les émollients, les silicones , les α-hydroxyacides, les agents anti-mousse, les agents hydratants, les vitamines, les parfums, les conservateurs, les tensioactifs, les charges, les séquestrants, les polymères, les propulseurs, les agents alcalinisants ou acidifiants, les colorants, ou tout autre ingrédient habituellement utilisé en cosmétique, en particulier pour la fabrication de compositions antisolaires sous forme d'émulsions.

Les corps gras peuvent être constitués par une huile ou une cire ou leurs mélanges, et ils comprennent également les acides gras, les alcools gras et les esters d'acides gras. Les huiles peuvent être choisies parmi les huiles animales, végétales, minérales ou de synthèse et notamment parmi l'huile de vaseline, l'huile de paraffine, les huiles de silicone, volatiles ou non, les isoparaffines, les poly-α-oléfines, les huiles fluorées et perfluorées. De même, les cires peuvent être choisies parmi les cires animales, fossiles, végétales, minérales ou de synthèse connues en soi.

Parmi les solvants organiques, on peut citer les alcools et polyols inférieurs.

Les épaississants peuvent être choisis notamment parmi les acides polyacryliques réticulés, les gommes de guar et celluloses modifiées ou non telles que la gomme de guar hydroxypropylée, la méthylhydroxyéthylcellulose, l'hydroxypropylméthyl cellulose ou encore l'hydroxyéthylcellulose.

Bien entendu, l'homme de l'art veillera à choisir ce ou ces éventuels composés complémentaires et/ou leurs quantités de manière telle que les propriétés avantageuses, en particulier l'effet de synergie, attachées intrinsèquement à l'association binaire conforme à l'invention ne soient pas, ou substantiellement pas, altérées par la ou les adjonctions envisagées.

Les compositions de l'invention peuvent être préparées selon les techniques bien connues de l'homme de l'art, en particulier celles destinées à la préparation d'émulsions de type huile-dans-eau ou eau-dans-huile.

Cette composition peut se présenter en particulier sous forme d'émulsion, simple ou complexe (H/E, E/H, H/E/H ou E/H/E) telle qu'une crème, un lait, un gel ou un gel crème, de poudre, de bâtonnet solide et éventuellement être conditionnée en aérosol et se présenter sous forme de mousse ou de spray.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR 2315991 et FR 2416008).

La composition cosmétique de l'invention peut être utilisée comme composition protectrice de l'épiderme humain ou des cheveux contre les rayons ultraviolets, comme composition antisolaire ou comme produit de maquillage.

La composition cosmétique de l'invention peut être également utilisée comme composition protectrice de la couleur naturelle ou des teintes artificielles des cheveux contre les effets néfastes des rayons ultraviolets.

Lorsque la composition cosmétique selon l'invention est utilisée pour la protection de l'épiderme humain contre les rayons UV, ou comme composition antisolaire, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, de bâtonnet solide, de stick, de mousse aérosol ou de spray.

Lorsque la composition cosmétique selon l'invention est utilisée pour la protection des cheveux, elle peut se présenter sous forme de shampooing, de lotion, de gel, d'émulsion, de dispersion vésiculaire non ionique, de laque pour cheveux et constituer par exemple une composition à rincer, à appliquer avant ou après shampooing, avant ou après i coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque la composition est utilisée comme produit de maquillage des cils, des sourcils ou de la peau, tel que crème de traitement de l'épiderme, fond de teint, bâton de rouge à lèvres, fard à paupières, fard à joues, mascara ou ligneur encore appelé "eye liner", elle peut se présenter sous forme solide ou pâteuse, anhydre ou aqueuse, comme des émulsions huile dans eau ou eau dans huile, des dispersions vésiculaires non ioniques ou encore des suspensions.

A titre indicatif, pour les formulations antisolaires conformes à l'invention qui présentent un support de type émulsion huile-dans-eau, la phase aqueuse (comprenant notamment les filtres hydrophiles) représente généralement de 50 à 95% en poids, de préférence de 70 à 90% en poids, par rapport à l'ensemble de la formulation, la phase huileuse (comprenant notamment les filtres lipophiles) de 5 à 50% en poids, de préférence de 10 à 30% en poids, par rapport à l'ensemble de la formulation, et le ou les (co)émulsionnant(s) de 0,5 à 20% en poids, de préférence de 2 à 10% en poids, par rapport à l'ensemble de la formulation.

Un autre objet de la présente invention réside dans un procédé de traitement cosmétique de la peau ou des cheveux destiné à les protéger contre les effets des rayons UV consistant à appliquer sur ceux-ci une quantité efficace d'une composition cosmétique telle que définie ci-dessus.

Un autre objet de la présente invention réside dans un procédé de traitement cosmétique des cheveux destiné protéger leur couleur naturelle ou leur coloration artificielle contre les effets des rayons UV consistant à appliquer sur ceux-ci une quantité efficace d'une composition cosmétique telle que définie ci-dessus.

Enfin, un autre objet de l'invention consiste en une utilisation d'une composition contenant l'association spécifique de filtres UV telle que définie précédemment comme agent photoprotecteur dans et pour la fabrication de compositions cosmétiques ou dermopharmaceutiques.

Des exemples concrets, mais nullement limitatifs, illustrant l'invention, vont maintenant être donnés.

### EXEMPLES :

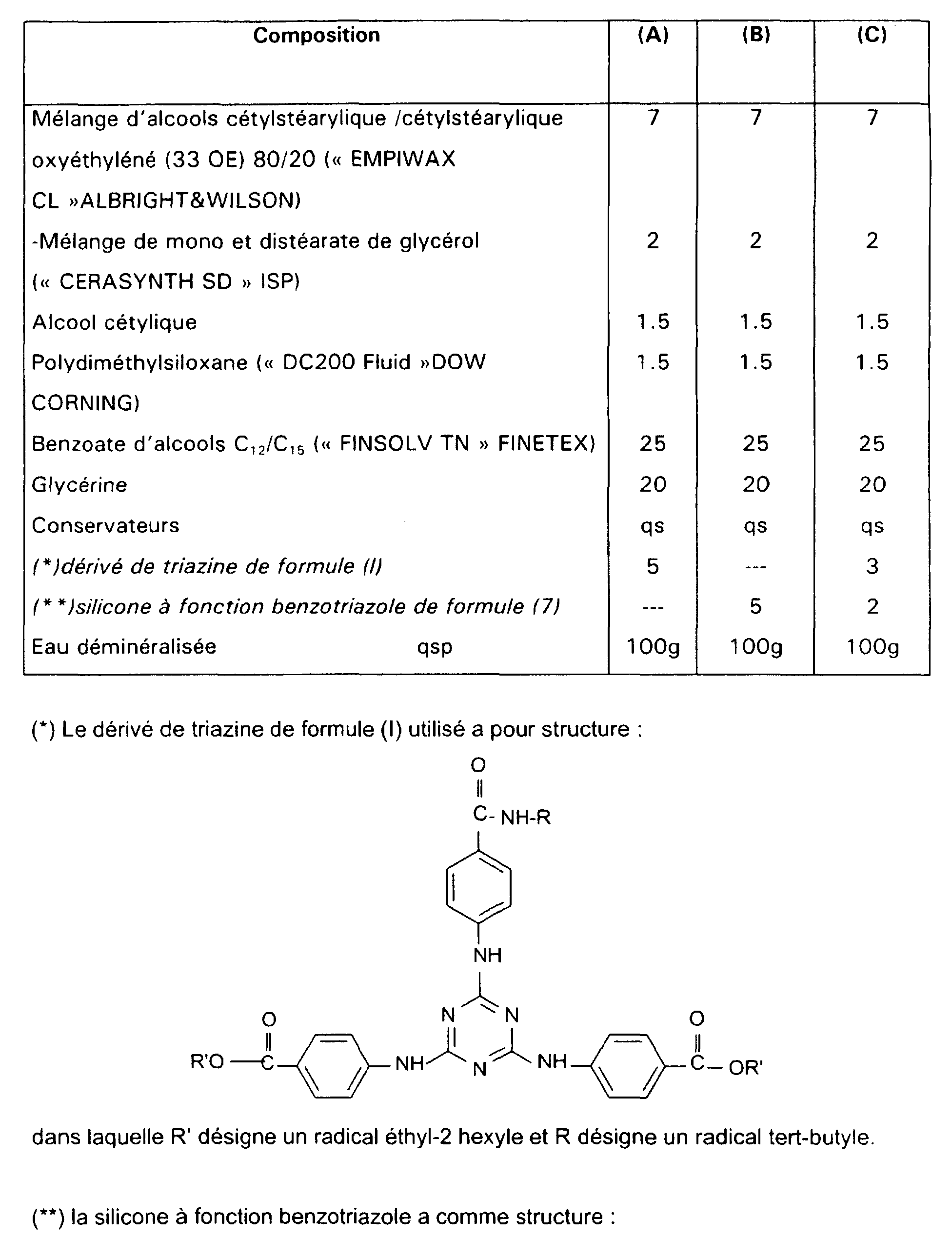

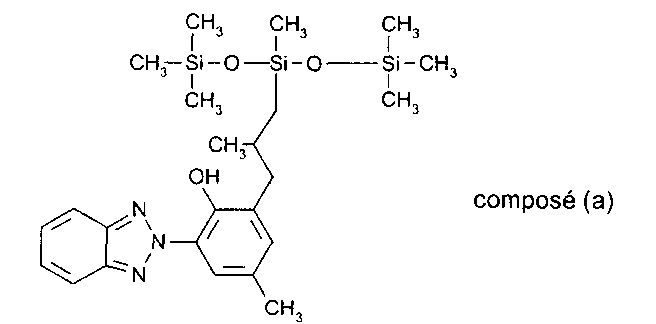

Pour chacune de ces formulations (A), (B), et (C), on a ensuite déterminé le facteur de protection solaire (FPS) qui lui était attaché. Celui-ci a été déterminé en utilisant la méthode *in vitro* décrite par B.L. DIFFEY et al. dans J. Soc. Cosmet. Chem. 40-127-133 (1989) ; cette méthode consiste à déterminer les facteurs de protection monochromatiques tous les 5 nm dans une gamme de longueurs d'onde de 290 à 400 nm et à calculer à partir de ceux-ci le facteur de protection solaire selon une équation mathématique donnée.

Les compositions des différentes formulations étudiées et les résultats en facteur de protection solaire moyen (moyenne sur trois essais) obtenus sont rassemblés dans le tableau (I) donné ci-dessous.

**Tableau (I)**

| **Composition** | **SPF « in Vitro »** | **Ecart - type** |
|---|---|---|
| (A) (hors invention) | 6.8 | 0.6 |
| (B) (hors invention) | 5.2 | 1.0 |
| (C) (invention) | 9.7 | 0.8 |

Ces résultats montrent la synergie de l'association dérivé de triazine /silicone à fonction benzotriazole en terme d'efficacité de la protection solaire.

## Revendications

1. Composition cosmétique et/ou dermatologique comprenant, dans un support cosmétiquement et/ou dermatologiquement acceptable :
(i) à titre de premier filtre au moins un dérivé de 1,3,5-triazine de formule (I) suivante : 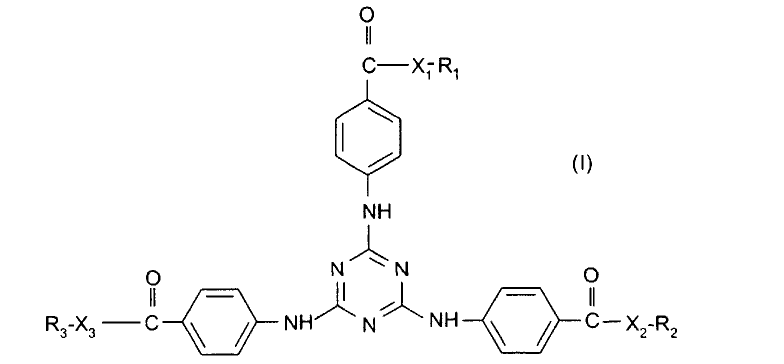 dans laquelle :
- X₁, X₂ et X₃, identiques ou différents, représentent l'oxygène ou un radical -NR-;
- les radicaux R, identiques ou différents, désignent l'hydrogène ou un radical alkyle en C₁-C₁₈, linéaire ou ramifié, un radical cycloalkyle en C₅-C₁₂ pouvant être substitué par un ou plusieurs radicaux alkyle en C₁-C₄ ;
- R₁, R₂ et R₃, identiques ou différents, sont choisis parmi : l'hydrogène ; un métal alcalin ; un radical ammonium éventuellement substitué par un ou plusieurs radicaux alkyle ou hydroxyalkyle ; un radical alkyle linéaire ou ramifié en C₁-C₁₈ ; un radical cycloalkyle en C₅-C₁₂ éventuellement substitué par un ou plusieurs radicaux alkyle en C₁-C₄ ; un radical polyoxyéthyléné comprenant de 1 à 6 unités d'oxyde d'éthylène et dont le groupe OH terminal est méthylé ; un radical de formule (II), (III) ou (IV) suivantes : 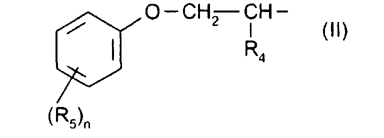 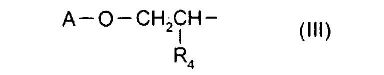 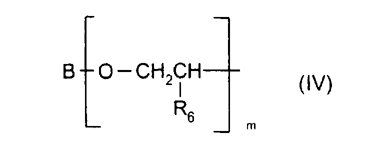 dans lesquelles :
- R₄ est l'hydrogène ou un radical méthyle ;
- R₅ est un radical alkyle en C₁-C₉ ;
- n est un nombre entier allant de 0 à 3 ;
- m est un nombre entier allant de 1 à 10 ;
- A est un radical alkyle en C₄-C₈ ou un radical cycloalkyle en C₅-C₈ ;
- B est choisi parmi : un radical alkyle linéaire ou ramifié en C₁-C₈ ; un radical cycloalkyle en C₅-C₈ ; un radical aryle éventuellement substitué par un ou plusieurs radicaux alkyle en C₁-C₄;
- R₆ est l'hydrogène ou un radical méthyle ;
(ii) à titre de deuxième filtre, au moins un dérivé silicié à fonction benzotriazole comprenant au moins une unité de formule (1) suivante :
O_{(3-a)/2}Si (R₇)ₐ-G (1)
dans laquelle :
- R₇ représente un radical alkyle en C₁-C₁₀ éventuellement halogéné ou un radical phényle ou un radical triméthylsilyloxy,
- a est un nombre entier choisi entre 0 et 3 inclusivement,
- et le symbole G désigne un radical monovalent lié directement à un atome de silicium, et qui répond à la formule (2) suivante : 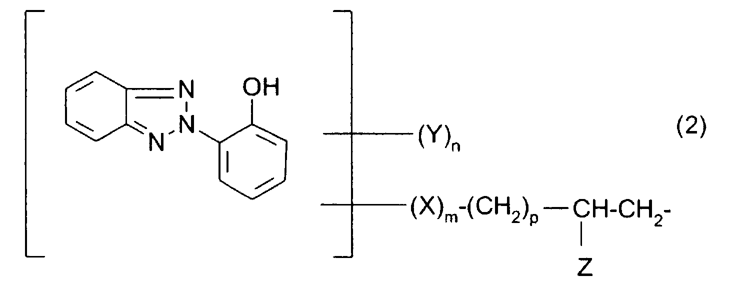 dans laquelle :
- Y, identiques ou différents, sont choisis parmi les radicaux alkyles en C₁-C₈, les halogènes et les radicaux alkoxy en C₁-C₄ étant entendu que, dans ce dernier cas, deux Y adjacents d'un même noyau aromatique peuvent former ensemble un groupement alkylidène dioxy dans lequel le groupe alkylidène contient de 1 à 2 atomes de carbone,
- X représente O ou NH,
- Z représente l'hydrogène ou un radical alkyle en C₁-C₄,
- n est un nombre entier compris entre 0 et 3 inclusivement,
- m est 0 ou 1,
- p représente un nombre entier compris entre 1 et 10, inclusivement; lesdits premier et second filtres étant présents dans lesdites compositions dans une quantité efficace pour produire une activité synergique au niveau des facteurs de protection solaire conférés.

2. Composition selon la revendication 1, où les dérivés de 1,3,5-triazine de formule (I) présentent l'ensemble des caractéristiques suivantes :
- X₁, X₂ et X₃ sont identiques et représentent l'oxygène ;
- R₁ est choisi parmi : un radical cycloalkyle en C₅-C₁₂ éventuellement substitué par un ou plusieurs radicaux alkyles en C₁-C₄; un radical de formule (II), (III) ou (IV) ci-dessus dans lesquelles :
- B est un radical alkyle en C₁-C₄;
- R₆ est le radical méthyle;
- R₂ et R₃, identiques ou différents, sont choisis parmi : l'hydrogène ; un métal alcalin ; un radical ammonium éventuellement substitué par un ou plusieurs radicaux alkyle ou hydroxyalkyle ; un radical alkyle linéaire ou ramifié en C₁-C₁₈ ; un radical cycloalkyle en C₅-C₁₂ éventuellement substitué par un ou plusieurs radicaux alkyle en C₁-C₄ ; un radical de formule (Il), (III) ou (IV) ci-dessus dans lesquelles :
- B est un radical alkyle en C₁-C₄;
- R₆ est le radical méthyle.

3. Composition selon la revendication 1, où les dérivés de 1,3,5-triazine de formule (I) présentent l'ensemble des caractéristiques suivantes :
- X₁ est l'oxygène ; X₂ est le radical -NH- ou l'oxygène ;
- X₃ est le radical -NH- ;
- R₃ est choisi parmi : un radical alkyle linéaire ou ramifié en C₁-C₁₈ ; un radical cycloalkyle en C₅-C₁₂ éventuellement substitué avec un ou plusieurs radicaux alkyles en C₁-C₄ ;
- R₁ est choisi parmi : l'hydrogène ; un métal alcalin ; un radical ammonium ; un radical de formule (IV) ; un radical alkyle linéaire ou ramifié en C₁-C₁₈ ; un radical cycloalkyle en C₅-C₁₂ éventuellement substitué avec un ou plusieurs radicaux alkyles en C₁-C₄ ;
- si X₂ est le radical -NH-, alors R₂ est choisi parmi : un radical alkyle linéaire ou ramifié en C₁-C₁₈ ; un radical cycloalkyle en C₅-C₁₂ éventuellement substitué avec un ou plusieurs radicaux alkyles en C₁-C₄ ;
- si X₂ est l'oxygène, alors R₂ est choisi parmi l'hydrogène ; un métal alcalin ; un radical ammonium ; un radical de formule (IV) ; un radical alkyle linéaire ou ramifié en C₁-C₁₈ ; un radical cycloalkyle en C₅-C₁₂ éventuellement substitué avec un ou plusieurs radicaux alkyles en C₁-C₄.

4. Composition selon la revendication 1, où les dérivés de 1,3,5-triazine de formule (I) présentent l'ensemble des caractéristiques suivantes :
- X₁, X₂ et X₃ désignent simultanément -NR- ;
- les radicaux R, identiques ou différents, désignent l'hydrogène ou un radical alkyle en C₁-C₁₈, linéaire ou ramifié, un radical cycloalkyle en C₅-C₁₂ pouvant être substitué par un ou plusieurs radicaux alkyle en C₁-C₄ ;
- R₁, R₂ et R₃, identiques ou différents, désignent l'hydrogène ou un radical R .

5. Composition selon la revendication 1, où le dérivé de 1,3,5-triazine répond à la formule suivante : 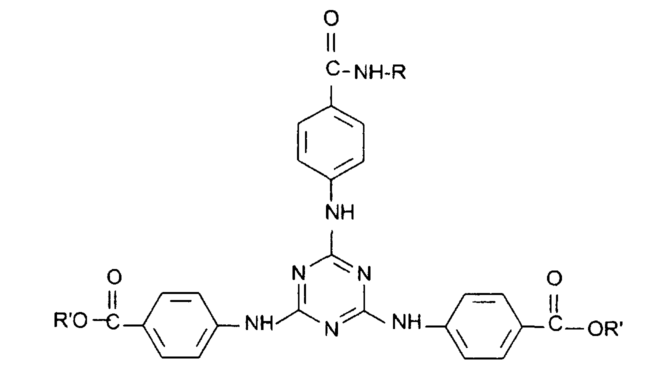 dans laquelle R' désigne un radical éthyl-2 hexyle et R désigne un radical tert-butyle.

6. Composition selon la revendication 1, où le dérivé de 1,3,5-triazine répond à la formule suivante : 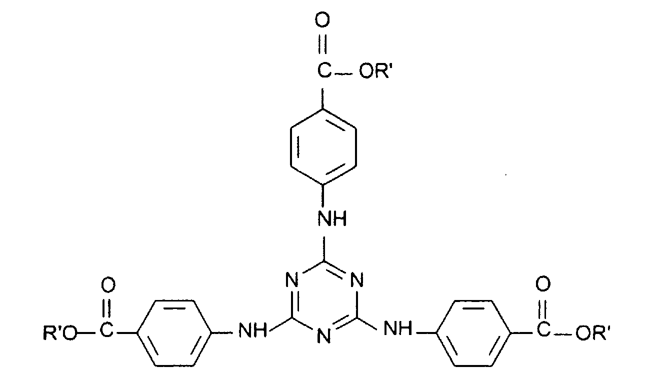 dans laquelle R' désigne un radical 2-éthyl hexyle.

7. Composition selon l'une quelconque des revendications 1 à 6, où les dérivés de 1,3,5-triazine sont présents à une teneur allant de 0,5 % à 20 %, de préférence de 1 % à 10 % en poids, par rapport au poids total de la composition.

8. Composition selon l'une quelconque des revendications 1 à 7, caractérisée par le fait que le dérivé silicié à fonction benzotriazole répond à l'une des formules (5) ou (6) suivantes : 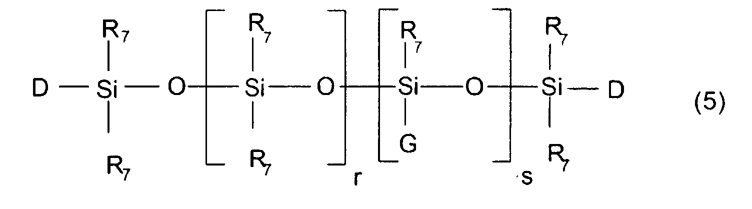 ou 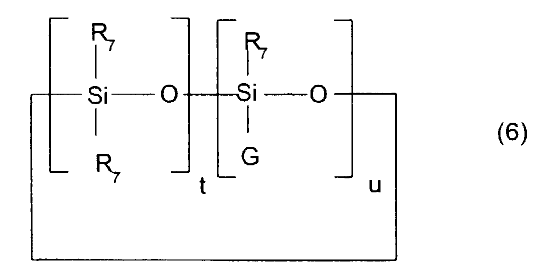 dans lesquelles :
- R₇, identiques ou différents, sont choisis parmi les radicaux alkyles en C₁-C₁₀, phényle, trifluoro-3,3,3 propyle et triméthylsilyloxy, au moins 80% en nombre des radicaux R₇ étant méthyle,
- D, identiques ou différents sont choisis parmi les radicaux R₇ et le radical G,
- r est un nombre entier compris entre 0 et 50 inclusivement, et s est un nombre entier compris entre 0 et 20 inclusivement, et si s = 0, au moins l'un des deux symboles D désigne G,
- u est un nombre entier compris entre 1 et 6 inclusivement, et t est un nombre entier compris entre 0 et 10 inclusivement, étant entendu que t + u est égal ou supérieur à 3,
- et le symbole G répond à la formule (2) définie dans la revendication 1.

9. Composition selon la revendication 8, caractérisée par le fait que la silicone benzotriazole répond à la formule (7) suivante : 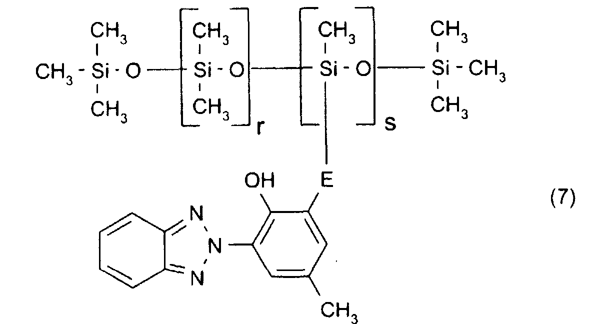 avec
0 ≤ r ≤ 10,
1 ≤ s ≤ 10,
et où E représente le radical divalent :

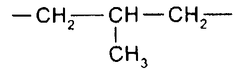

10. Composition selon la revendication 9, caractérisée par le fait que le dérivé silicié à fonction benzotriazole répond à la formule suivante

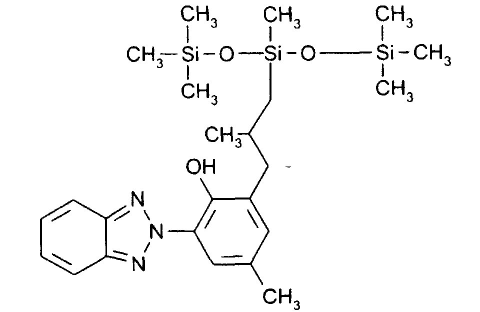

11. Composition selon l'une quelconque des revendications 1 à 10, caractérisée par le fait que les dérivés siliciés à fonction benzotriazole sont présents à une teneur allant de 0,5 % à 20 %, de préférence de 1 % à 10 % en poids, par rapport au poids total de la composition.

12. Composition selon l'une quelconque des revendications 1 à 11, caractérisée par le fait que ledit support cosmétiquement acceptable se présente sous la forme d'une émulsion de type huile-dans-eau.

13. Composition selon l'une quelconque des revendications 1 à 12, caractérisée par le fait qu'elle comprend en outre un ou plusieurs filtres organiques complémentaires actifs dans l'UV-A et/ou UV-B, hydrophiles ou lipophiles.

14. Composition selon l'une quelconque des revendications 1 à 14, caractérisée par le fait qu'elle comprend en outre, à titre d'agents photoprotecteurs complémentaires, des pigments ou des nanopigments d'oxydes métalliques, enrobés ou non, capables de bloquer physiquement, par diffusion et/ou réflection, le rayonnement UV.

15. Composition selon la revendication 14, caractérisée par le fait que lesdits pigments ou nanopigments sont choisis parmi les oxydes de titane, de zinc, de fer, de zirconium, de cérium et leurs mélanges, enrobés ou non.

16. Composition selon l'une quelconque des revendications 1 à 15, caractérisée par le fait qu'elle comprend en outre au moins un agent de bronzage et/ou de brunissage artificiel de la peau.

17. Composition selon l'une quelconque des revendications 1 à 16, caractérisée par le fait qu'il s'agit d'une composition destinée à la protection des cheveux contre les rayons ultraviolets.

18. Utilisation d'une composition définie à l'une quelconque des revendications 1 à 17, comme, ou pour la fabrication de, compositions cosmétiques pour la protection de la peau et/ou des cheveux contre le rayonnement ultraviolet, en particulier le rayonnement solaire.

19. Utilisation d'une composition définie à l'une quelconque des revendications 1 à 17 comme agent photoprotecteur dans et pour la fabrication de compositions cosmétiques ou dermopharmaceutiques.

20. Utilisation d'une composition définie à l'une quelconque des revendications 1 à 17 comme, ou pour la fabrication de, compositions cosmétiques pour la protection de la couleur naturelle ou artificielle des cheveux contre les effets des rayons UV, en particulier le rayonnement solaire.

21. Procédé de traitement cosmétique pour protéger la peau et/ou les cheveux contre le rayonnement ultraviolet, en particulier le rayonnement solaire, caractérisé en ce qu'il consiste à appliquer sur ceux-ci une quantité efficace d'une composition telle que définie à l'une quelconque des revendications 1 à 17.

22. Procédé de traitement cosmétique des cheveux destiné à protéger leur couleur naturelle ou leur coloration artificielle contre les effets des rayons UV consistant à appliquer sur ceux-ci une quantité efficace d'une composition que telle que définie à l'une quelconque des revendications 1 à 17.

## Patentansprüche

1. Kosmetische und/oder dermatologische Zusammensetzung, die in einem kosmetisch und/oder dermatologisch akzeptablen Träger enthält:
(i) als erstes Filter mindestens ein 1,3,5-Triazinderivat der folgenden Formel (I): 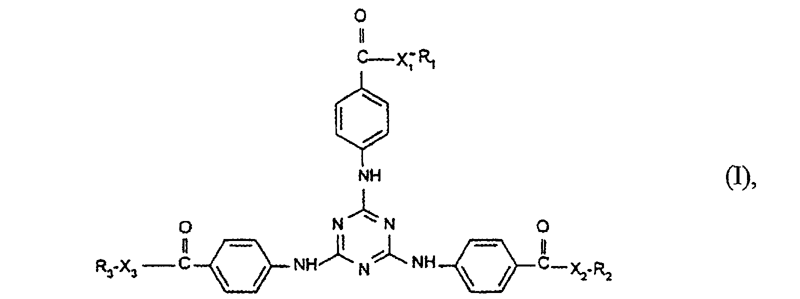 worin:
- die Gruppen X₁, X₂ und X₃, die identisch oder voneinander verschieden sind, Sauerstoff oder die Gruppe -NR- bedeuten;
- die Gruppen R, die identisch oder voneinander verschieden sind, Wasserstoff, eine geradkettige oder verzweigte C₁₋₁₈-Alkylgruppe oder eine C₅₋₁₂-Cycloalkylgruppe bedeuten, die mit einer oder mehreren C₁₋₄-Alkylgruppen substituiert sein kann;
- die Gruppen R₁, R₂ und R₃, die identisch oder voneinander verschieden sind, ausgewählt sind unter: Wasserstoff; einem Alkalimetall; einer Ammoniumgruppe, die gegebenenfalls mit einer oder mehreren Alkyl- oder Hydroxyalkylgruppen substituiert ist; einer geradkettigen oder verzweigten C₁₋₁₈-Alkylgruppe; einer C₅-₁₂-Cycloalkylgruppe, die gegebenenfalls mit einer oder mehreren C₁₋₄-Alkylgruppen substituiert ist; einer Polyoxyethylen-haltige Gruppe, die 1 bis 6 Ethylenoxideinheiten aufweist und deren OH-Endgruppe methyliert ist; einer Gruppe der folgenden Formeln (II), (III) oder (IV): 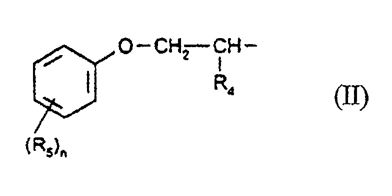 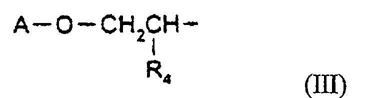 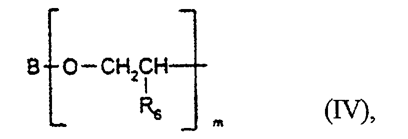 worin bedeuten:
- R₄ Wasserstoff oder Methyl;
- R₅ eine C₁₋₉-Alkylgruppe;
- n Null oder eine ganze Zahl im Bereich von 1 bis 3;
- m eine ganze Zahl im Bereich von 1 bis 10;
- A eine C₄₋₈-Alkylgruppe oder eine C₅₋₈-Cycloalkylgruppe;
- B eine geradkettige oder verzweigte C₁₋₈-Alkylgruppe; eine C₅₋₈-Cycloalkylgruppe; eine Arylgruppe, die gegebenenfalls mit einer oder mehreren C₁₋₄-Alkylgruppen substituiert ist; und
- R₆ Wasserstoff oder Methyl.
und ii) als zweites Filter ein siliciumhaltiges Derivat mit Benzotriazolgruppe, das mindestens eine Einheit der folgenden Formel (1) enthält:
O_{(3-a)/2}Si(R₇)ₐ-G (1),
worin bedeuten:
- R₇ eine C₁₋₁₀-Alkylgruppe, die gegebenenfalls halogeniert ist, eine Phenylgruppe oder eine Trimethylsilyloxygruppe,
- a 0 oder eine ganze Zahl im Bereich von 1 bis 3, und
- G eine einwertige Gruppe, die direkt an ein Siliciumatom gebunden ist und der folgenden Formel (2) entspricht: 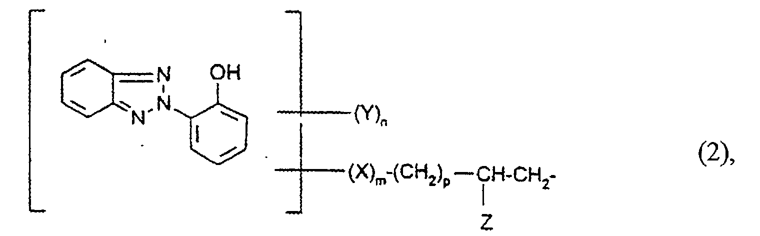 worin:
- die Gruppen Y, die identisch oder voneinander verschieden sind, unter den C₁₋₈-Alkylgruppen, Halogenen und C₁₋₄-Alkoxygruppen ausgewählt sind, wobei im letzten Fall zwei angrenzende Gruppen Y an dem gleichen aromatischen Ring auch gemeinsam eine Alkylidendioxygruppe bilden können, worin die Alkylidengruppe 1 bis 2 Kohlenstoffatome aufweist,
- X O oder NH bedeutet,
- Z Wasserstoff oder eine C₁₋₄-Alkylgruppe bedeutet,
- n 0 oder eine ganze Zahl im Bereich von 1 bis 3 ist,
- m 0 oder 1 ist, und
- p eine ganze Zahl im Bereich von 1 bis 10 bedeutet,
wobei das erste und das zweite Filter in den Zusammensetzungen in Mengenanteilen vorliegen, die ausreichend sind, um einen synergistischen Effekt bezüglich des Lichtschutzfaktors hervorzurufen.

2. Zusammensetzung nach Anspruch 1, wobei das 1,3,5-Triazinderivat der Formel (I) die gesamten folgenden Eigenschaften aufweist:
- X₁, X₂ und X₃ sind identisch und bedeuten Sauerstoff;
- R₁ ist ausgewählt unter: einer C₅₋₁₂-Cycloalkylgruppe, die gegebenenfalls mit einer oder mehreren C₁₋₄-Alkylgruppen substituiert ist; einer oben definierten Gruppe der Formel (II), (III) oder (IV), worin bedeuten:
- B eine C₁₋₄-Alkylgruppe; und
- R₆ die Methylgruppe;
- die Gruppen R₂ und R₃, die identisch oder verschieden sind, sind ausgewählt unter: Wasserstoff; einem Alkalimetall; einer Ammoniumgruppe, die gegebenenfalls mit einer oder mehreren Alkyl- oder Hydroxyalkylgruppen substituiert ist; einer geradkettigen oder verzweigten C₁₋₁₈-Alkylgruppe; einer C₅₋₁₂-Cycloalkylgruppe, die gegebenenfalls mit einer oder mehreren C₁₋₄-Alkylgruppen substituiert ist; einer Gruppe der oben definierten Formel (II), (III) oder (IV), worin bedeuten:
- B eine C₁₋₄-Alkylgruppe; und
- R₆ die Methylgruppe.

3. Zusammensetzung nach Anspruch 1, wobei das 1,3,5-Triazinderivat der Formel (I) die gesamten folgenden Eigenschaften aufweist:
- X₁ bedeutet Sauerstoff; X₂ bedeutet -NH- oder Sauerstoff;
- X₃ bedeutet die Gruppe -NH-;
- R₃ ist ausgewählt unter: einer geradkettigen oder verzweigten C₁₋₁₈-Alkylgruppe; einer C₅₋₁₂-Cycloalkylgruppe, die gegebenenfalls mit einer oder mehreren C₁₋₄-Alkylgruppen substituiert ist;
- R₁ ist ausgewählt unter: Wasserstoff; einem Alkalimetall; einer Ammoniumgruppe; einer Gruppe der Formel (IV); einer geradkettigen oder verzweigten C₁₋₁₈-Alkylgruppe; einer C₅₋₁₂-Cycloalkylgruppe, die gegebenenfalls mit einer oder mehreren C₁₋₄-Alkylgruppen substituiert ist;
- wenn X₂ die Gruppe -NH- bedeutet, dann ist R₂ ausgewählt unter einer geradkettigen oder verzweigten C₁₋₁₈-Alkylgruppe; einer C₅₋₁₂-Cycloalkylgruppe, die gegebenenfalls mit einer oder mehreren C₁₋₄-Alkylgruppen substituiert ist;
- wenn X₂ Sauerstoff bedeutet, dann ist R₂ ausgewählt unter Wasserstoff; einem Alkalimetall; einer Ammoniumgruppe; einer Gruppe der Formel (IV); einer geradkettigen oder verzweigten C₁₋₁₈-Alkylgruppe; einer C₅₋₁₂-Cycloalkylgruppe, die gegebenenfalls mit einer oder mehreren C₁₋₄-Alkylgruppen substituiert ist.

4. Zusammensetzung nach Anspruch 1, wobei das 1,3,5-Triazinderivat der Formel (I) die gesamten folgenden Eigenschaften aufweist:
- X₁, X₂ und X₃ sind identisch und bedeuten -NR-;
- die Gruppen R, die identisch oder voneinander verschieden sind, bedeuten Wasserstoff, eine geradkettige oder verzweigte C₁₋₁₈-Alkylgruppe; oder eine C₅₋₁₂-Cycloalkylgruppe, die mit einer oder mehreren C₁₋₄-Alkylgruppen substituiert sein kann.
- die Gruppen R₁, R₂ und R₃, die identisch oder voneinander verschieden sind, bedeuten Wasserstoff oder eine Gruppe R.

5. Zusammensetzung nach Anspruch 1, wobei das 1,3,5-Triazinderivat der folgenden Formel entspricht: 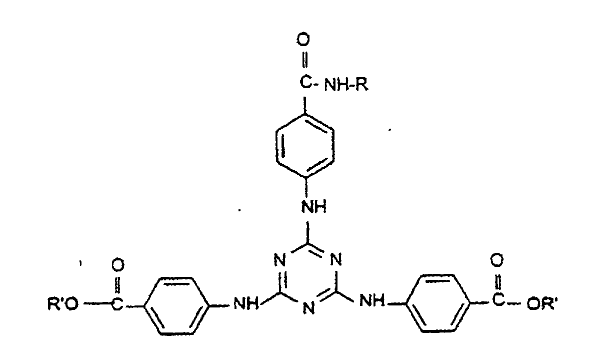 worin die Gruppen R' die 2-Ethylhexylgruppe und die Gruppe R die *tert*.-Butylgruppe bedeuten.

6. Zusammensetzung nach Anspruch 1, wobei das 1,3,5-Triazinderivat der folgenden Formel entspricht: 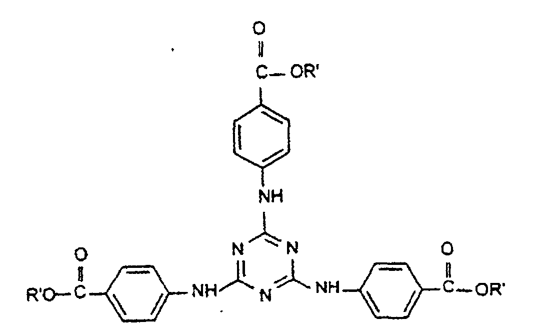 worin die Gruppen R' die 2-Ethylhexylgruppe bedeuten.

7. Zusammensetzung nach einem der Ansprüche 1 bis 6, wobei die 1,3,5-Triazinderivate in einem Mengenanteil von 0,5 bis 20 Gew.-% und vorzugsweise von 1 bis 10 Gew.-%, bezogen auf das Gesamtgewicht der Zusammensetzung, vorliegen.

8. Zusammensetzung nach einem der Ansprüche 1 bis 7, dadurch gekennzeichnet, daß das siliciumhaltige Derivat mit Benzotriazolgruppe einer der folgenden Formeln (5) oder (6) entspricht: 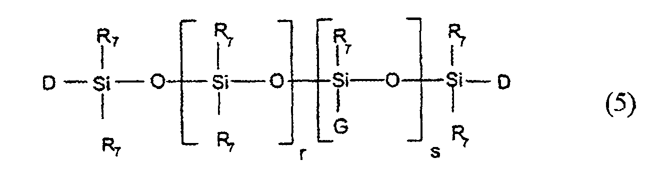 oder 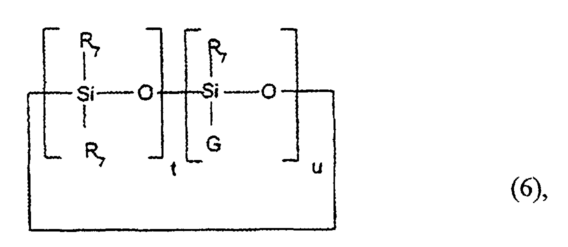 worin:
- die Gruppen R₇, die identisch oder voneinander verschieden sind, unter den C₁₋₁₀-Alkylgruppen, Phenyl, 3,3,3-Trifluorpropyl und Trimethylsilyloxy ausgewählt sind, wobei mindestens 80 % der Anzahl der Gruppen R7 Methyl bedeutet,
- die Gruppen D, die identisch oder voneinander verschieden sind, unter den Gruppen R₇ und der Gruppe G ausgewählt sind,
- r 0 oder eine ganze Zahl im Bereich von 1 bis 50 und s 0 oder eine ganze Zahl im Bereich von 1 bis 20 bedeutet, und mindestens eine der beiden Gruppen D G bedeutet, wenn s 0 ist,
- u eine ganze Zahl im Bereich von 1 bis 6 und t 0 oder eine ganze Zahl im Bereich von 1 bis 10 ist, mit der Maßgabe, daß t + u 3 bedeutet oder darüber liegt, und
- die Gruppe G der in Anspruch 1 definierten Formel (2) entspricht.

9. Zusammensetzung nach Anspruch 8, dadurch gekennzeichnet, daß Benzotriazolsilicon der folgenden Formel (7) entspricht: 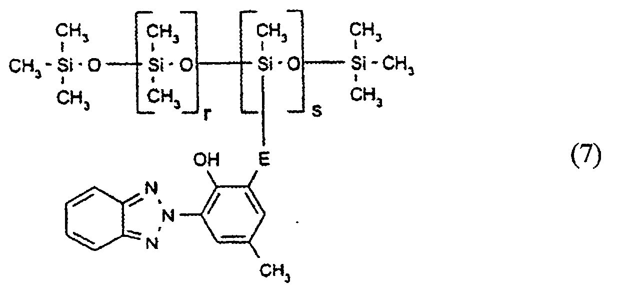 worin bedeuten:
0 ≤ r ≤ 10,
1 ≤ s ≤ 10,
und E die zweiwertige Gruppe:

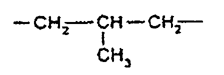

10. Zusammensetzung nach Anspruch 9, dadurch gekennzeichnet, daß Benzotriazolsilicon der folgenden Formel entspricht:

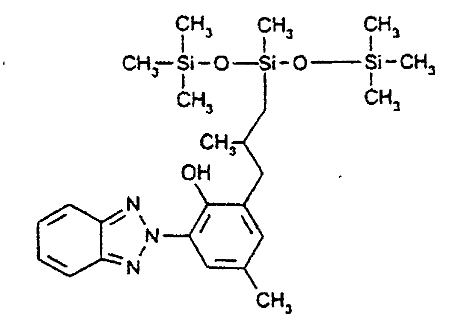

11. Zusammensetzung nach einem der Ansprüche 1 bis 10, dadurch gekennzeichnet, daß die siliciumhaltigen Derivate mit Benzotriazolgruppe in einem Mengenanteil von 0,5 bis 20 Gew.-% und vorzugsweise von 1 bis 10 Gew.-%, bezogen auf das Gesamtgewicht der Zusammensetzung, vorliegen.

12. Zusammensetzung nach einem der Ansprüche 1 bis 11, dadurch gekennzeichnet, daß der kosmetisch akzeptable Träger in Form einer Öl-in-Wasser-Emulsion vorliegt.

13. Zusammensetzung nach einem der Ansprüche 1 bis 12, dadurch gekennzeichnet, daß sie ferner mindestens ein oder mehrere zusätzliche, herkömmliche, lipophile und/oder hydrophile, im UV-A- und/oder UV-B-Bereich wirksame organische Filter enthält.

14. Zusammensetzung nach einem der Ansprüche 1 bis 14, dadurch gekennzeichnet, daß sie als zusätzliche Lichtschutzmittel ferner Pigmente oder Nanopigmente von Metalloxiden enthalten, die umhüllt oder nicht umhüllt sind, und die UV-Strahlung durch Streuung und/oder Reflexion pysikalisch sperren können.

15. Zusammensetzung nach Anspruch 14, dadurch gekennzeichnet, daß die ggf. umhüllten Pigmente und Nanopigmente unter den Oxiden von Titan, Zink, Eisen, Zirconium und Cer und deren Gemischen ausgewählt sind.

16. Zusammensetzung nach einem der Ansprüche 1 bis 15, dadurch gekennzeichnet, daß sie ferner mindestens ein Mittel zur Bräunung und/oder künstlichen Braunfärbung der Haut enthält.

17. Zusammensetzung nach einem der Ansprüche 1 bis 16, dadurch gekennzeichnet, daß es sich um eine Zusammensetzung zum Schutz der Haare gegen UV-Strahlung handelt.

18. Verwendung einer Zusammensetzung nach einem der Ansprüche 1 bis 17 als kosmetische Zusammensetzung zum Schutz der Haut und/oder der Haare gegen UV-Strahlung und insbesondere Sonnenlicht oder zu deren Herstellung.

19. Verwendung einer Zusammensetzung nach einem der Ansprüche 1 bis 17 als Lichtschutzmittel in einer kosmetischen oder dermopharmazeutischen Zusammensetzung oder zu deren Herstellung.

20. Verwendung einer Zusammensetzung nach einem der Ansprüche 1 bis 17 als kosmetische Zusammensetzung zum Schutz der natürlichen oder künstlichen Haarfarbe gegen die Wirkungen der UV-Strahlung und insbesondere Sonnenlicht oder zu deren Herstellung.

21. Kosmetisches Verfahren zum Schutz der Haut und/oder der Haare gegen UV-Strahlung und insbesondere Sonnenlicht, dadurch gekennzeichnet, daß es darin besteht, auf diese eine wirksame Menge einer Zusammensetzung nach einem der Ansprüche 1 bis 17 aufzutragen.

22. Kosmetisches Verfahren zur Behandlung der Haare zum Schutz der natürlichen oder künstlichen Haarfarbe gegen die Wirkungen der UV-Strahlung, das darin besteht, auf diese eine wirksame Menge einer Zusammensetzung nach einem der Ansprüche 1 bis 17 aufzutragen.

## Claims

1. Cosmetic and/or dermatological composition comprising, in a cosmetically and/or dermatologically acceptable support:
(i) as first screening agent, at least one 1,3,5-triazine derivative of formula (I) below: 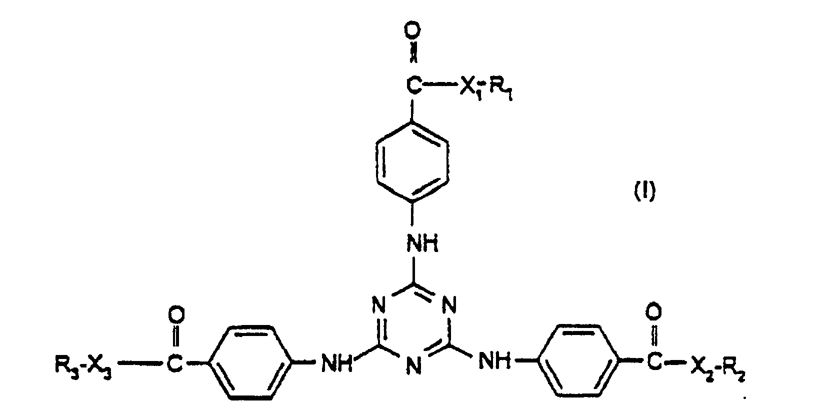 in which:
- X₁, X₂ and X₃, which may be identical or different, represent oxygen or a radical -NR-;
- the radicals R, which may be identical or different, denote hydrogen or a linear or branched C₁-C₁₈ alkyl radical or a C₅-C₁₂ cycloalkyl radical which may be substituted with one or more C₁-C₄ alkyl radicals;
- R₁, R₂ and R₃, which may be identical or different, are chosen from: hydrogen; an alkali metal; an ammonium radical optionally substituted with one or more alkyl or hydroxyalkyl radicals; a linear or branched C₁-C₁₈ alkyl radical; a C₅-C₁₂ cycloalkyl radical optionally substituted with one or more C₁-C₄ alkyl radicals; a polyoxyethylenated radical comprising from 1 to 6 ethylene oxide units and in which the end OH group is methylated; a radical of formula (II), (III) or (IV) below: 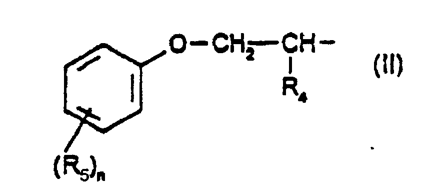 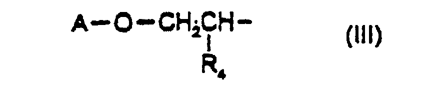 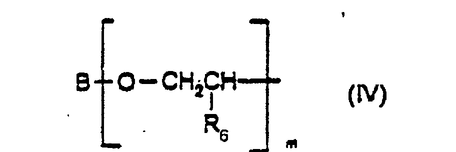 in which:
- R4 is hydrogen or a methyl radical;
- R₅ is a C₁-C₉ alkyl radical;
- n is an integer ranging from 0 to 3;
- m is an integer ranging from 1 to 10;
- A is a C₄-C₈ alkyl radical or a C₅-C₈ cycloalkyl radical;
- B is chosen from: a linear or branched C₁-C₈ alkyl radical; a C₅-C₈ cycloalkyl radical; an aryl radical optionally substituted with one or more C₁-C₄ alkyl radicals;
- R₆ is hydrogen or a methyl radical;
(ii) as second screening agent, at least one silicon derivative containing a benzotriazole function, comprising at least one unit of formula (1) below:
O_{(3-a)/2} Si (R₇)ₐ - G (1)
in which:
- R₇ represents an optionally halogenated C₁-C₁₀ alkyl radical or a phenyl radical or a trimethylsilyloxy radical,
- a is an integer chosen between 0 and 3 inclusive, and
- the symbol G denotes a monovalent radical linked directly to a silicon atom, and which corresponds to formula (2) below: 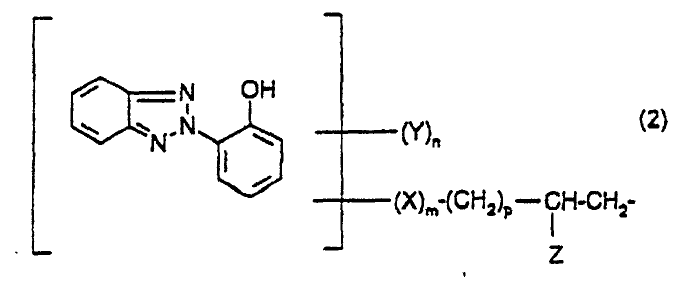 in which:
- Y, which may be identical or different, are chosen from C₁-C₉ alkyl radicals, halogens and C₁-C₄ alkoxy radicals, it being understood that, in the latter case, two adjacent groups Y on the same aromatic ring can together form an alkylidenedioxy group in which the alkylidene group contains 1 or 2 carbon atoms,
- X represents O or NH,
- Z represents hydrogen or a C₁-C₄ alkyl radical,
- n is an integer between 0 and 3 inclusive,
- m is 0 or 1,
- p represents an integer between 1 and 10 inclusive; the said first and second screening agents being present in the said compositions in an amount which is effective for producing synergistic activity with regard to the sun protection factors imparted.

2. Composition according to Claim 1, in which the 1,3,5-triazine derivatives of formula (I) have all of the following characteristics:
- X₁, X₂ and X₃ are identical and represent oxygen;
- R₁ is chosen from: a C₅-C₁₂ cycloalkyl radical optionally substituted with one or more C₁-C₄ alkyl radicals; a radical of formula (II), (III) or (IV) above in which:
- B is a C₁-C₄ alkyl radical;
- R₆ is a methyl radical;
- R₂ and R₃, which may be identical or different, are chosen from: hydrogen; an alkali metal; an ammonium radical optionally substituted with one or more alkyl or hydroxyalkyl radicals; a linear or branched C₁-C₁₈ alkyl radical; a C₅-C₁₂ cycloalkyl radical optionally substituted with one or more C₁-C₄ alkyl radicals; a radical of formula (II), (III) or (IV) above in which:
- B is a C₁-C₄ alkyl radical;
- R₆ is a methyl radical.

3. Composition according to Claim 1, in which the 1,3,5-triazine derivatives of formula (I) have all of the following characteristics:
- X₁ is oxygen; X₂ is an -NH- radical or oxygen;
- X₃ is an -NH- radical;
- R₃ is chosen from: a linear or branched C₁-C₁₈ alkyl radical; a C₅-C₁₂ cycloalkyl radical optionally substituted with one or more C₁-C₄ alkyl radicals;
- R₁ is chosen from: hydrogen; an alkali metal; an ammonium radical; a radical of formula (IV); a linear or branched C₁-C₁₈ alkyl radical; a C₅-C₁₂ cycloalkyl radical optionally substituted with one or more C₁-C₄ alkyl radicals;
- if X₂ is an -NH- radical, then R2 is chosen from: a linear or branched C₁-C₁₈ alkyl radical; a C₅-C₁₂ cycloalkyl radical optionally substituted with one or more C₁-C₄ alkyl radicals;
- if X₂ is oxygen, then R₂ is chosen from: hydrogen; an alkali metal; an ammonium radical; a radical of formula (IV); a linear or branched C₁-C₁₈ alkyl radical; a C₅-C₁₂ cycloalkyl radical optionally substituted with one or more C₁-C₄ alkyl radicals.

4. Composition according to Claim 1, in which the 1,3,5-triazine derivatives of formula (I) have all of the following characteristics:
- X₁, X₂ and X₃ simultaneously denote -NR-;
- the radicals R, which may be identical or different, denote hydrogen or a linear or branched C₁-C₁₈ alkyl radical or a C₅-C₁₂ cycloalkyl radical which may be substituted with one or more C₁-C₄ alkyl radicals;
- R₁, R₂ and R₃, which may be identical or different, denote hydrogen or a radical R.

5. Composition according to Claim 1, in which the 1,3,5-triazine derivative corresponds to the following formula: 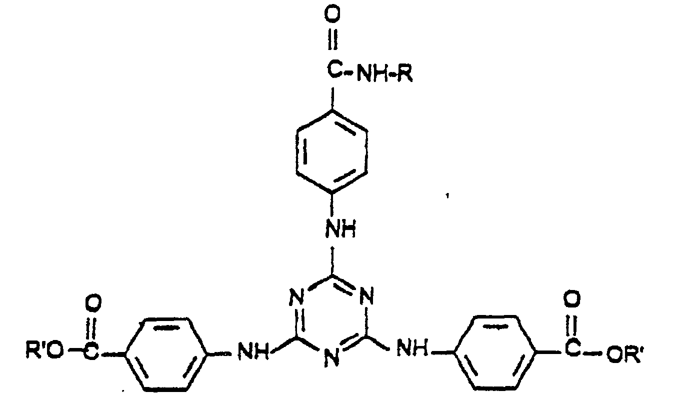 in which R' denotes a 2-ethylhexyl radical and R denotes a tert-butyl radical.

6. Composition according to Claim 1, in which the 1,3,5-triazine derivative corresponds to the following formula: 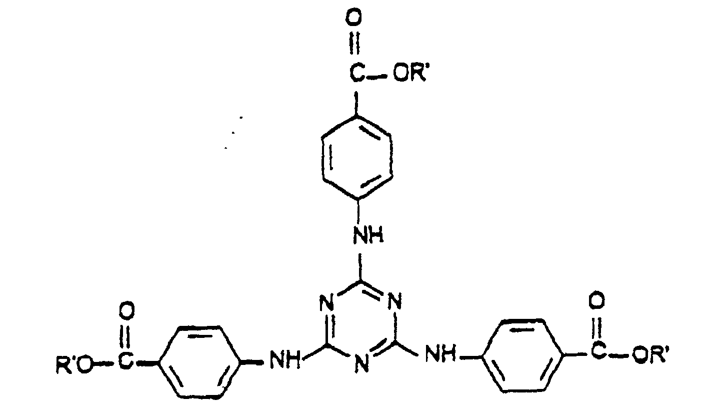 in which R' denotes a 2-ethylhexyl radical.

7. Composition according to any one of Claims 1 to 6, in which the 1,3,5-triazine derivatives are present in a content ranging from 0.5% to 20%, preferably from 1% to 10%, by weight, relative to the total weight of the composition.

8. Composition according to any one of Claims 1 to 7, characterized in that the silicon derivative containing a benzotriazole function corresponds to one of the formulae (5) or (6) below: 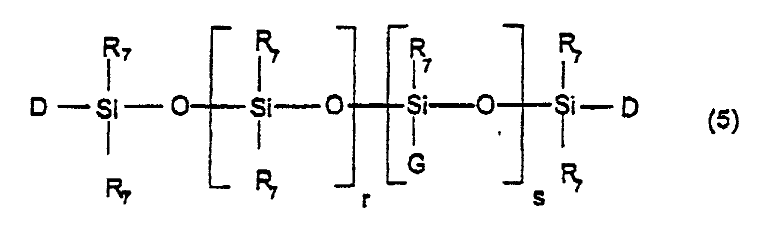 or 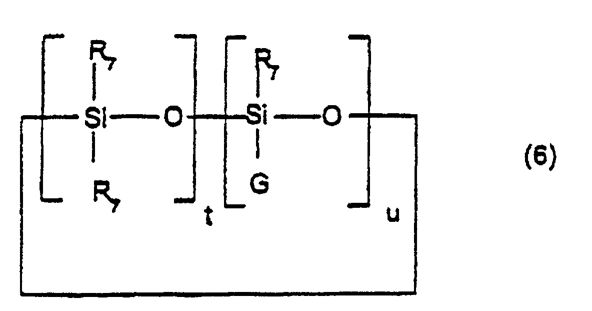 in which:
- R₇, which may be identical or different, are chosen from C₁-C₁₀ alkyl, phenyl, 3,3,3-trifluoropropyl and trimethylsilyloxy radicals, at least 80%, in numerical terms, of the radicals R₇ being methyl,
- D, which may be identical or different, are chosen from the radicals R₇ and the radical G,
- r is an integer between 0 and 50 inclusive, and s is an integer between 0 and 20 inclusive, and if s = 0, at least one of the two symbols D denotes G,
- u is an integer between 1 and 6 inclusive, and t is an integer between 0 and 10 inclusive, it being understood that t + u is equal to or greater than 3, and
- the symbol G corresponds to formula (2) defined in Claim 1.

9. Composition according to Claim 8, characterized in that the benzotriazole silicone compound corresponds to formula (7) below 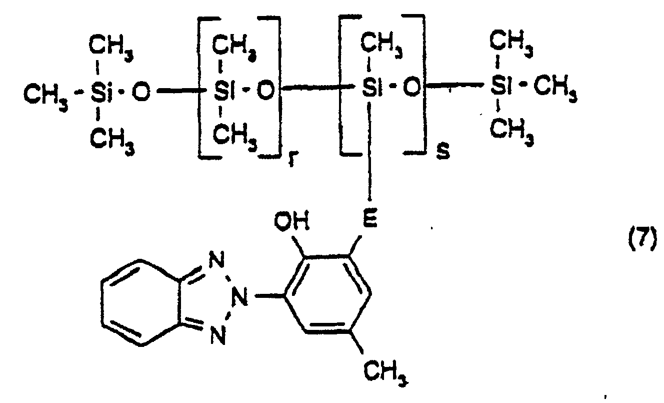 with
0 ≤ r ≤ 10,
1 ≤ s ≤ 10,
and in which E represents the divalent radical:

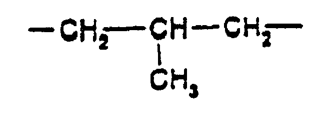

10. Composition according to Claim 9, characterized in that the silicon derivative containing a benzotriazole function corresponds to the following formula:

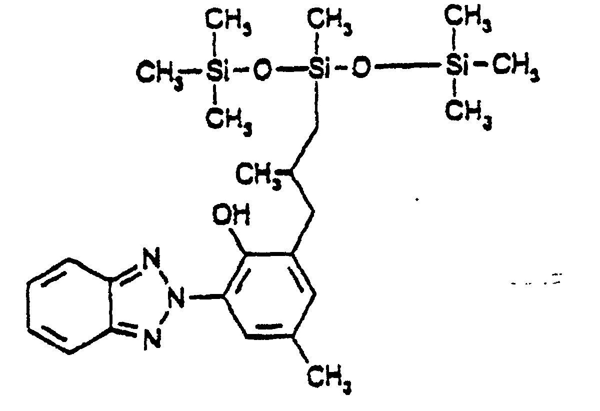

11. Composition according to any one of Claims 1 to 10, characterized in that the silicon derivatives containing a benzotriazole function are present in a content ranging from 0.5% to 20%, preferably from 1% to 10%, by weight, relative to the total weight of the composition.

12. Composition according to any one of Claims 1 to 11, characterized in that the said cosmetically acceptable support is in the form of an emulsion of oil-in-water type.

13. Composition according to any one of Claims 1 to 12, characterized in that it also comprises one or more additional hydrophilic or lipophilic organic screening agents which are active in the UV-A and/or UV-B range.

14. Composition according to any one of Claims 1 to 13, characterized in that it also. comprises, as additional photoprotective agents, pigments or nanopigments of coated or uncoated metal oxides, capable of physically blocking UV radiation, by scattering and/or reflection.

15. Composition according to Claim 14, characterized in that the said pigments or nanopigments are chosen from titanium oxide, zinc oxide, iron oxide, zirconium oxide and cerium oxide and mixtures thereof, which may be coated or uncoated.

16. Composition according to any one of Claims 1 to 15, characterized in that it also comprises at least one agent for artificially tanning and/or browning the skin.

17. Composition according to any one of Claims 1 to 16, characterized in that it is a composition intended to protect the hair against ultraviolet rays.

18. Use of a composition defined in any one of Claims 1 to 17, as, or for the manufacture of, cosmetic compositions for protecting the skin and/or the hair against ultraviolet radiation, in particular solar radiation.

19. Use of a composition defined in any one of Claims 1 to 17, as a photoprotective agent in, and for the manufacture of, cosmetic or dermopharmaceutical compositions.

20. Use of a composition defined in any one of Claims 1 to 17, as, or for the manufacture of, cosmetic compositions for protecting the natural or artificial colour of the hair against the effects of UV rays, in particular solar radiation.

21. Cosmetic treatment process for protecting the skin and/or the hair against ultraviolet radiation, in particular solar radiation, characterized in that it consists in applying an effective amount of a composition as defined in any one of Claims 1 to 17 to the skin and/or the hair.

22. Cosmetic treatment process for the hair which is intended to protect its natural colour or its artificial coloration against the effects of UV rays, this process consisting in applying an effective amount of a composition as defined in any one of Claims 1 to 17 to the hair.
